# EUROPEAN PATENT APPLICATION

(11) **EP 0 850 616 A1**
(43) Date of publication of application: **01.07.1998**
(21) Application number: 96120569.7
(22) Date of filing: 20.12.1996
(51) Int. Cl.: A61F 13/15

(54) **A dry laid structure comprising odour control means**

(71) Applicant: THE PROCTER & GAMBLE COMPANY, Cincinnati, Ohio 45202 (US)
(72) Inventor: Gagliardi, Ivano, 65123 Pescara (IT); Carlucci, Giovanni, 66100 CHieti (IT)
(74) Representative: Hirsch, Uwe Thomas

(57) **Abstract**

The present invention relates to a dry laid fibrous structure for providing odour control. The structure comprises a dry laid fibrous web and a latex coating on at least one surface of the web and further comprises an odour control means for controlling malodours. The structure can be an absorbent structure for further providing absorption of fluids

## Description

### FIELD OF THE INVENTION

The present invention relates to dry laid fibrous structures for providing odour control and, optionally, for absorbing fluids. The structures contain odour control means and are bonded by application of a liquid binder; they are particularly suitable for use in disposable absorbent articles.

### BACKGROUND OF THE INVENTION

Fibrous structures, particularly fibrous structures for absorbing fluids, are manufactured for many uses, for example they are incorporated into absorbent articles such as disposable diapers, incontinent pads and catamenial napkins as fluid absorption or fluid transmission and/or diffusion elements, for example, as absorbent cores that are intended to absorb and retain body fluids. Fibrous structures, and more specifically fibrous structures used in absorbent articles as fluid absorption or fluid transmission and/or diffusion elements, usually comprise a multiplicity of components so as to improve their specific performances; further components can be also included to provide the structure with added benefits.

Whilst the primary focus of absorbent articles remains the ability of these articles to absorb and retain fluids, another important area of development in this field is in fact the control of odorous compounds contained within the absorbed fluids or their degradation products. There are a wide range of compounds which may be present in an absorbent article during use which result in the formation of malodorous. These compounds include fatty acids, ammonia, amines, sulphur containing compounds and ketones and aldehydes.

The literature shows many references relating to odour control in products such as diapers, catamenials or incontinent pads, and various odour control means comprising different odour control agents are described for use in absorbent articles in order to address the problem of malodour formation. These agents are available in different forms, e.g. as water-insoluble particulate material, or as water-soluble compounds. Accordingly, odour control means can be incorporated in absorbent articles in many ways, being typically comprised into a fibrous structure of the absorbent article, e.g. at least partially into the absorbent core, for example by spraying a solution of a suitable water soluble odour control compound onto the fibrous structure, or, alternatively, by distributing an odour control means in form of particulate material within the fibrous structure.

Interactions between odour control means and components constituting the absorbent article, and, more specifically, constituting the fibrous structure that comprises the odour control means, are therefore possible, and sometimes such interactions can decrease the effectiveness of the odour control means.

Dry laying and, more specifically, air laying processes are widely used to produce webs from dry fibres, which can in turn be used e.g. as structures for absorbing fluids. Particularly, dry laying refers to the formation of carded webs, i.e., webs in which the fibres are oriented (carded) in a given direction, whereas the air laying process refers to the formation of webs with a completely random fibre orientation; the properties of such air laid webs are therefore somewhat isotropic. The fibrous webs produced by dry laying processes are sort, flexible and porous, and are particularly suitable for use as liquid absorbent structures in absorbent articles, such as disposable diapers, sanitary napkins, incontinent pads, and wipes.

The dry laid manufacturing process generally comprises a web formation and layering step and a web bonding and stabilizing step; in dry laying processes in fact the fibres, that can be of any type, e.g. cellulosic, synthetic, or any combination thereof, are formed or condensed into a web, but such web lacks integrity, and must therefore be stabilized. Different techniques for bonding and stabilizing a dry formed web are known in the art, i.e. mechanical, thermal and chemical bonding processes. Bonding a web structure by means of a chemical is one of the most common methods of bonding in the nonwoven industry, and consists in the application of a chemical binder to the web and in the curing of the binder. The most widely used chemical is latex, since it is cheap, versatile, easy to apply, and very effective as a binder. Several methods are known to apply the latex binder to the fibrous web, while spray bonding and print bonding are particularly preferred for fibrous webs intended to be used in absorbent articles.

Methods for incorporating further components in a dry laid fibrous web are also known in the art; particularly, in US Patent No. 4,765,780 a process and apparatus is described for forming air laid fibrous webs having a multiplicity of components, such as a two layer absorbent core with one layer having an absorbent gelling material in particle form homogeneously blended therein, the other layer being substantially free of absorbent gelling material particles. Similar techniques can be used for incorporating in a dry laid fibrous web different types of components, e.g. an odour control means in particle form in order to provide the absorbent structure constituted by the dry laid fibrous web with the further benefit of odour control.

In European application EP-A-463 716 a dry laid absorbent structure is described that comprises a fibrous web with an absorbent gelling material therein and is stabilized by application of a latex coating to at least a surface of the web. Although the coating imparts integrity to the resulting structures without impairing the effectiveness of the absorbent gelling material, such structures are not suitable for providing odour control, which is a particularly important feature in structures that are intended for use in absorbent articles.

The latex binder is usually an aqueous emulsion, and can be applied to one or both major flat surfaces of the fibrous web, but negative interaction can occur between the latex composition and the odour control means, particularly when the odour control means comprises one or more odour control agents in particle form, since the latex binder can generally create a coating on odour control particles and therefore impair at least partially their effectiveness.

Problems created by interaction between a latex binder and an odour control means, particularly in particle form, are somewhat different from those involved in the combined use of such a binder and of an absorbent gelling material in a unitary structure. Without being bound to any particular theory, the liquid absorption mechanism of absorbent gelling material is based on swelling and acts by means of internal diffusion of liquid. Therefore, a particle of absorbent gelling material partially coated by a latex binder can still acquire fluid from the non-coated portion and diffuse it internally, being still capable of swelling at a reduced rate. Odour control agents on the other hand, particularly those in particle form, usually cannot relate to such an internal diffusion mechanism and their performances are strictly related to the surface that is actually available for the interaction between the agent and the odorous substances. Even a partial coating by a latex composition therefore effectively reduces the odour control capacity of odour control agents in substantial terms. This effect can be particularly relevant when an adsorption mechanism against gaseous odorous compounds is involved in the odour control action of some odour control agents, as it is the case e.g. for activated carbon and zeolites.

Hence, there still exists a need for a dry laid fibrous structure, preferably a dry laid absorbent fibrous structure, stabilized by means of a latex binder and capable of providing odour control. It has now been discovered that this need can be addressed by a dry laid fibrous structure stabilized by means of a latex binder and comprising an odour control means, in which the negative interaction between the odour control means and the latex binder are avoided or at least reduced, therefore providing an absorbent structure which is soft and has a good integrity, while also featuring odour control.

### SUMMARY OF THE INVENTION

The present invention relates to a dry laid fibrous structure for providing odour control and, optionally, for absorbing fluids, comprising a dry laid fibrous web having a first surface and a second surface aligned approximately opposite to the first surface; the fibrous structure comprises a latex coating on at least one of the surfaces, and further comprises an odour control means for controlling malodour.

### BRIEF DESCRIPTION OF THE DRAWINGS

While the specification concludes with claims particularly pointing out and distinctly claiming the present invention, it is believed that the present invention will be better understood from the following description in conjunction with the following drawings:
FIG. 1 is a schematic, fragmentary side elevational view of an apparatus for making a fibrous structure according to the present invention;
FIG. 2 is an enlarged, cross-sectional view of a fibrous structure according to the present invention;
FIG. 3 is an enlarged, cross-sectional view of an alternate embodiment of a fibrous structure according to the present invention.

### DETAILED DESCRIPTION OF THE INVENTION

The present invention relates to a dry laid fibrous structure for providing odour control; preferably, the dry laid fibrous structure of the present invention is also intended for absorption of fluids and for controlling of the odours associated with the absorbed fluids. The structure is constituted by a dry laid fibrous web bonded by means of a liquid binder, preferably a latex binder, and comprises an odour control means. In a preferred embodiment, the structures of the present invention are incorporated as absorbent structures into absorbent articles, preferably as absorbent cores, and are intended to absorb and retain the various body fluids. Absorbent articles, and more specifically disposable absorbent articles, refer to articles such as sanitary napkins, disposable diapers, incontinent pads, that are worn by a user adjacent to the body and are intended to absorb and contain the various body fluids that are discharged from the body (e.g., vaginal discharges, menses, sweat, and/or urine) and which is intended to be discarded after a single use.

Disposable absorbent articles typically comprise a fluid pervious topsheet, a fluid impervious backsheet, that can optionally be water vapour and/or gas pervious, and an absorbent element comprised therebetween.

The term "use", as used herein, refers to the period of time that starts when the absorbent article is actually put in contact with the anatomy of the user.

The development of unpleasant odours from body fluids as sweat, menstrual blood, vaginal discharges, or urine, are basically due to two different causes: a) malodorous chemical compounds already contained in the body fluids; and b) malodorous chemical compounds produced by the bacterial metabolism when the bacteria come in contact with body fluids for a prolonged period of time.

Means for controlling the odours associated with body fluids and their incorporation in disposable absorbent articles are widely known in the art, and various odour-control agents have been disclosed in the literature.

Different classes of odour-control agents are known in the art according to their different mechanisms of action. Disposable absorbent articles can comprise only one odour-control agent, or combinations of various odour-control agents, optionally belonging to different classes and therefore performing different actions for the control of unpleasant odours associated with body fluids.

A first class of odour-control agents is constituted by those compounds that interfere with the bacterial metabolism, in order to avoid or to reduce the production of malodorous metabolites from the body fluids; such agents can be bactericides or bacteriostats and are typically available as water-soluble compounds.

A second class of odour-control agents comprises those compounds, typically in particulate form, that are capable of adsorbing within their structure the odoriferous substances, both those already present in the body fluids as such and those produced by the bacterial metabolism.

Another class of odour-control agents comprises perfumes that essentially mask the unpleasant odours; moisture-activated encapsulated perfume particles can also be used, in which the perfumes are released only when the material is wetted, to provide their action during the use of the product, and to optionally avoid interaction with other odour-absorbing agents before the product is used, if such a combination is actually used as the odour-control means.

Any suitable odour-control agent known in the art can be incorporated in the dry laid fibrous structures of the present invention, to provide the structure with the benefit of odour control, e.g. towards those odours associated with absorbed body fluids when the dry laid fibrous structure is further capable of absorbing fluids and is preferably incorporated into disposable absorbent articles.

Suitable odour-control agents that can be employed in the practice of the present invention can be for example water-soluble antibacterial compounds. Such compounds include, for example, halogenated phenylene compounds (U.S. Patent 3,093,546), periodic acids (U.S. Patent 3,804,094), various copper compounds, especially copper acetate (U.S. Patent 4,385,632), various quaternary ammonium salts, which are well known for their antibacterial properties, e.g. cetyl pyridinium chloride, and the like. Alternatively, antibacterial compounds can be used conjointly with various particulate materials which, in use and in the presence of moisture, release the antibacterial agent. Zeolite materials, such as zeolites which are bactericidal by virtue of having absorbed therein and thereon various bactericidal cations such as copper, silver and zinc, can be advantageously used in the practice of this invention (U.S. Patent 4,525,410). In a preferred mode, the odour control agent is a water-insoluble particulate odour absorbing material such as chlorophyll particles, activated carbon granules, charcoal, ion exchange resin (Japanese 87019865), activated alumina, and absorbent zeolite materials, including the well known "molecular sieve" zeolites of the type A and X and the zeolite materials marketed under the trade name ABSCENTS by the Union Carbide Corporation and UOP, and which are typically available as a white powder in the 3-5 micron particle size range.

In a known mode, the odour-control agent can be a water-insoluble particulate odour-absorbing material such as chlorophyll particles, activated carbon granules, charcoal, ion exchange resin (Japanese 87019865), activated alumina, and absorbent zeolite materials, including the well known "molecular sieve" zeolites of the type A and X and the zeolite materials marketed under the trade name ABSCENTS by the Union Carbide Corporation and UOP, and which are typically available as a white powder in the 3-5 micron particle size range.

The odour-control agents used in the present invention can also comprise other compounds such as cyclodextrin, chelating agents, parabens, chitin, pH buffered materials, silica gel, clays, diatomaceous earth, polystyrene derivatives, starches, and the like. For example, chelating agents as those described in European Applications EP 96109178.2 and EP 96109179.0, both applications filed on 7 June 1996, are particularly preferred. Some partially neutralized hydrogel forming absorbent gelling materials, such as polyacrylate gelling material and acrylate grafted starch gelling material can be also used, preferably in combination with other odour-control agents.

Further odour control agents can comprise acidic compounds such as ascorbic acid, stearic acid, boric acid, maleic acid polymers, malonic acid, maleic acid, polyacrylic acid and monopotassium phosphate, or basic compounds such as inorganic salts of carbonates, bicarbonate, phosphate, biphosphate, sulfate, bisulfate, borate, and mixtures thereof, as those described in US 5037412, or as the combination of boric acid and sodium tetraborate described in International application WO 94/25077.

It is to be understood that the odour-control means employed in the practice of the present invention is not, simply, the odour-control agent, per se, added to the fibrous structure. Rather, the odour-control means comprises any combination of odour-control agents and, optionally, of other materials such as binders. Agglomerates of different odour control agents, e.g. with a binder, can therefore also be used, such as for example an agglomerate of zeolite and silica in particle form, as that described in European application EP 96109175.8, filed on 7 June 1996. The odour-control agent, on the other hand, is the specific odour-control compound.

Preferably, odour control agents are used in the present invention in particulate form and comprise those agents that act as adsorbers of gaseous odorous compounds. Particularly preferred are odour control means comprising zeolite, silica, preferably in form of silica gel, absorbent gelling material, and combinations thereof, such as the odour control means described in International application WO 95/26207, and in European applications EP 96109177.4, EP 96109173.3, EP 96109174.1, EP 96109176.6.

The fibrous structures of the present invention can be made using conventional equipment designed for dry laying processes, and although the invention is described hereinbelow with particular reference to air laid structures, it should be understood that other dry laying processes, e.g. carding, are also applicable.

The invention will be described as an air laid fibrous structure which is capable of providing absorption of aqueous fluids, particularly body fluids, and control of the odours associated with the absorbed fluids, being intended to be incorporated as absorbent structure in a disposable absorbent article, e.g. a sanitary napkin.

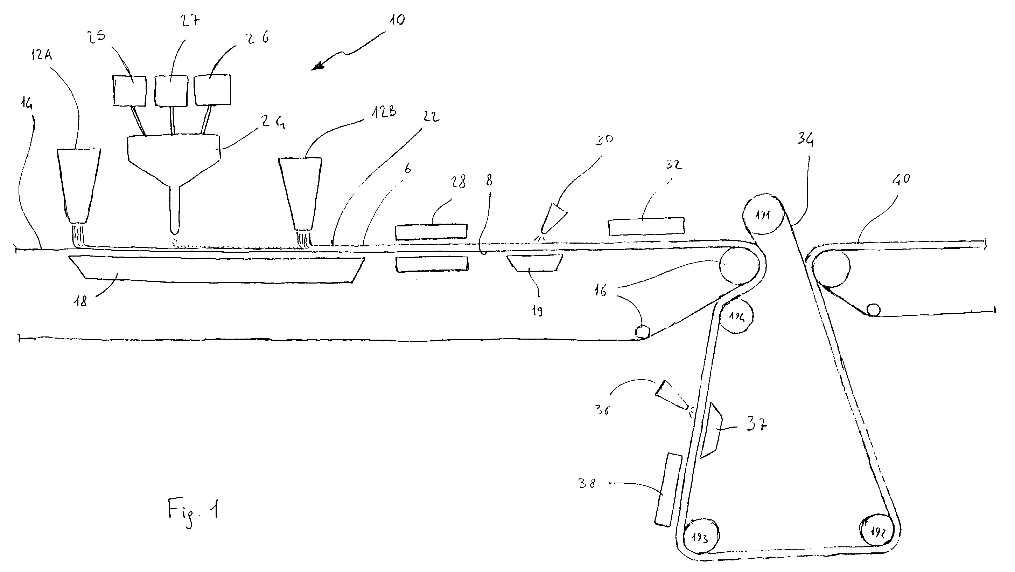

FIG. 1 is a simplified schematic illustration of a preferred embodiment for the manufacture of the fibrous structure of the present invention. In accordance with this embodiment, the air forming system, indicated generally by the numeral 10, includes a distributor unit 12 disposed transversely above a continuous forming screen 14 mounted on rollers 16 and driven by a suitable motor (not shown), and vacuum means or suction box 18 is positioned beneath the screen. In a conventional air forming system, upstream of the distributor unit is a defibrator or feeder (not shown), such as a hammermill or Rando-Feeder, where bales, laps or the like are defiberized, and further the fibres may be cleaned and/or blended if necessary or desired depending largely on the type of fibres used, the blend of fibres used, and the end product sought. For example, wood pulp fibres can be blended with synthetic fibres and applied as a blend by a single distributor, or different fibres can be each conveyed by a different distributor to the screen to form separate plies or layers.

The porous forming screen 14 is essentially coextensive with the distributors, and the suction box 18 beneath the screen draws the air stream downwardly and conveys the fibres to the surface of the screen thereby forming plies or a loose web 22. At this stage in the process, the web exhibits little integrity, and the vacuum means retains the loose, fibrous web on the screen. The web 22 has a first surface 6 that faces the distributor and a second surface 8, opposite to surface 6, that faces the forming screen 14.

It should be understood that the system may be modified to control the composition and thickness of the end product. For example, the distributor unit can comprise a plurality of individual distributors, and although FIG. 1 shows schematically two distributors at 12A and 12B, this number of distributors and particular arrangement can be altered or varied depending on such factors as machine speed, capacity, type of fibres, and end product desired.

Web 22 formed on screen 14 has incorporated therein an odour control means constituted by one or more odour control agents in particle or powder form. In a preferred embodiment as shown in FIG. 1, a dosing unit or feed hopper 24, containing particles 26 of the odour control means, or, more briefly, the odour control particles 26, is positioned in the middle of the distributor unit, i.e. between distributors 12A and 12B. In this manner, odour control particles 26 are deposited between plies of fibres laid by each distributor. That is, the odour control particles 26 are discharged from hopper 24 onto the moving layer of fibres laid down by distributor 12A, and the ply of fibres laid down by distributor 12B is laid over the odour control particles 26. It should be understood, however, that the plies are relatively porous, and therefore the odour control particles 26 may tend to migrate somewhat into adjacent pies. Where desired, the odour control particles may be blended with the fibres in one or more distributors, such as in distributor 12A or 12B, thereby forming a web with odour control particles intermixed with one or more fibrous plies of the web. It is however preferred that the odour control particles are distributed within the thickness of the web 22, intermediate the first and second surfaces 6, 8.

At this stage of the process, the web 22 condensed on forming wire 14 has very little integrity and requires stabilization. The web is advanced by the continuous screen, and if desired, the web first may be passed between compression rollers (not shown), which may be heated, to densify the web, but this step is optional. This densification step enhances the penetration of the latex into the web, and the degree or percent of densification can vary depending on such factors as the amount of odour control particles, basis weight of the web, the desired degree of penetration of the latex into the web, and the end product sought.

From there, the web is transported to a suitable dispensing means 30, such as a spray nozzle, doctor blade, roller applicator, or the like, where a latex binder is applied to the first surface 6 of the loose web 22. A vacuum applied by the same suction box 18 positioned beneath the dispensing means and screen helps to draw the latex into the web. The dispensing means or applicator is essentially coextensive with the width of the web, and preferably a substantially uniform coating is applied to the web surface. However, the latex may be applied as a non uniform, random or pattern coating, and because the latex is water-based, it will diffuse throughout the web and function as a binder when cured.

The latex when cured imparts integrity to the web, and therefore some penetration of the latex is required. The extent or degree of penetration of the latex into the web is controlled by controlling the amount of latex applied and by controlling the vacuum applied to the web in that the vacuum helps to draw the latex into the web. The latex is usually applied as an aqueous emulsion, and is a thermosetting plastic. In order to activate the latex, the latex emulsion contains a suitable curing agent or cross-linking agent, and after the web is coated, the latex is cured to effect cross-linking. Most typically, curing is accomplished by passing the coated web through a hot air oven or through an air drier 32, and the temperature typically ranges from about 100 °C to 260 °C, but this depends upon the specific type of latex resin used, upon the curing agent or cross-linking agent, upon the amount of latex, the thickness of the web, the degree of vacuum, and the machine speed.

It is desirable to coat the second surface 8 of the web 22 with latex as well, and this is readily accomplished by the dispensing means 36 as the web 22 is conveyed on the second screen 34 operating about pulleys 191, 192, 193, and 194. The second dispensing means 36 includes a suction box 37. This second latex coating is likewise cured by passing the web through a second oven 38 within about the same temperature range.

The resulting absorbent fibrous structure 40 exiting from the last oven now exhibits sufficient integrity and can be cut, rolled, packaged, etc.

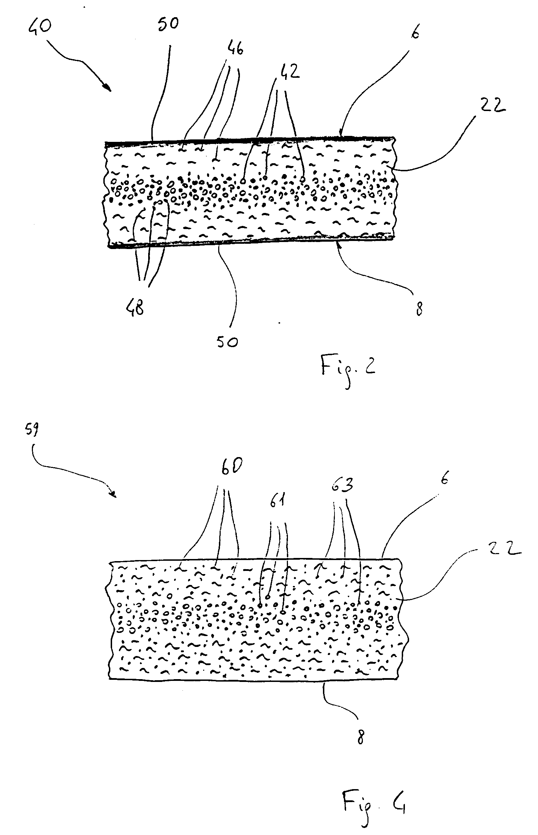

The absorbent structure 40 made in accordance with the foregoing process is illustrated in FIG. 2. The absorbent structure, indicated generally by the numeral 44, comprises a web 22 of randomly distributed fibres 46, such as wood pulp fibres, and odour control particles 26 are distributed in the web 22. It will be observed that the odour control particles are more concentrated in the middle zone of the web 22 that is intermediate the first and second surfaces 6 and 8, but some particles migrate to other sections of the web 22. Both first and second surfaces 6 and 8 of the web 22 bear a latex coating 50, indicated in the drawing by a shading, which has penetrated or impregnated the web 22 to some degree and has partially coated some of the fibres and odour control particles. As explained above, the penetration is controlled so as not to substantially impair the odour control capacity of the odour control particles.

Notwithstanding the latex coating, the fibrous structure is soft yet strong and absorbent, exhibiting a relatively high tensile strength. It is desirable for preferred absorbent fibrous structures of this type to have relatively low bulk, because a more dense absorbent structure, when compared to similar structures containing no latex and of about equal absorptive capacity but of higher bulk, can be thinner yet highly absorbent and consequently less bulky. A reduction in bulk, which means a reduction in volume the absorbent fibrous structure is occupying, without sacrificing significantly other desired properties is important from the standpoint of manufacturing, storage and packaging. Hence, for products of this invention the basis weight ranges from about 50 g/m² to 600 g/m², preferably from about 75 g/m² to 400 g/m², and more preferably from 250 g/m² to 350 g/m². There can be manufacturing constraints in producing a fibrous structure having a basis weight lower than about 50 g/m² in that such a fibrous structure may lack desired strength. When the basis weight exceeds the upper limit, the product may be too stiff and therefore not useful for most applications.

Any of a variety of fibres, including a blend or admixture, can be used in the fibrous structure of this invention. The fibres may be cellulosic, modified cellulosic, or synthetic, and include such fibres as wood pulp, rayon, cotton, cellulose acetate, polyester, polyethylene, polypropylene, nylon, and the like. A fibrous structure comprising cellulosic fibres such as wood pulp fibres is particularly useful as an absorbent structure in such products as sanitary napkins, disposable diapers or wipes because the cellulose is liquid absorbent and therefore enhances the overall absorbency of the structure. Products of this type, i.e., fibrous structures that are also absorbent, also advantageously use a blend of cellulosic and synthetic fibres, typically comprising about 65% to 95% by weight of cellulosic fibres, and more preferably up to about 20% by weight of the synthetic fibres. The synthetic fibres, which can be provided in any length including staple length, can improve the strength of the structure. They can also be treated to make them hydrophilic, in order not to decrease the absorbent capacity of the preferred absorbent fibrous structure.

Preferred fibrous structures described so far comprise hydrophilic fibres and are also substantially absorbent towards aqueous fluids, being useful as absorbent structures in disposable absorbent articles. Dry laid fibrous structures according to the present invention can also comprise hydrophobic fibres only, e.g. synthetic fibres, being therefore capable of odour control without absorbing and retaining liquid. Such type of structures can be comprised in disposable absorbent articles as a liquid receiving and transmitting layer, e.g. as an acquisition layer comprised between the topsheet and the absorbent core, which is capable of acquiring body fluid quickly and of transmitting it to the absorbent core, performing at the same time its odour control action towards the fluid. A structure comprising hydrophobic fibres only and being capable of acquiring and transmitting fluid without substantially retaining it could be advantageous in circumstances where retention of acquired liquid could clog the odour control means impairing its effectiveness. A structure according to the present invention comprising hydrophobic synthetic fibres only can also be useful in different applications, e.g. as a filter medium.

Thus, the type of fibres and the particular blend can be varied depending upon the end product. In addition to the foregoing uses, the absorbent structures of this invention can be suitably used for incontinent pads, diaper core, diaper insert, and for surgical and wound bandages, providing odour control and, preferably, absorbent capacity.

The odour control means included in the fibrous structure of the present invention can comprise a wide variety of odour control agents, in order to control unpleasant odours associated with the fluids that can contact them.

In their preferred use as absorbent cores in disposable absorbent articles the fibrous structures of the present invention are intended to absorb body fluids.

Various body fluids contain malodorous chemical compounds including acyclic and cyclic amines, aldehydes, fatty acids, and sulfur containing compounds such as sulfides. For example vaginal discharges and used sanitary napkins can contain many malodorous chemical compounds, for example, trimethylamine, pyridine, furaldehyde, isovaleric acid, and methyl mercaptan. The particular malodorous compounds which will be absorbed by various absorbent articles will vary depending upon the person who is wearing the absorbent article and the type of body fluid absorbed, i.e., urine, menstrual fluid, vaginal discharges, perspiration, milk, etc. For feminine pads, such as sanitary napkins or pantiliners, the length of time the article is worn, the quantity of fluid which is absorbed and the exposure of the pad to different body fluids will determine which odours can be emitted by the absorbent article.

Any known odour-control agent or any combination thereof that can be suitably included in a disposable absorbent article, including other materials such as binders and/or substrates, can be comprised in the fibrous structure of the present invention as the odour control means. Preferably the odour control means is incorporated in the fibrous structures of the present invention in particle or powder form, but it can also be incorporated by different known means, e.g. sprayed as an aqueous solution onto the first fibrous ply laid down by the distributor 12A in the apparatus illustrated in FIG. 1.

A preferred odour control means can be a combination of silica gel, zeolite and absorbent gelling material particles. The weight ratio of absorbent gelling material to silica to zeolite is preferably in the range of from 1:5:1 to 1:1:5, preferably from 1:3:1 to 1:1:3, most preferably from 1:1:1 to 1:1.5:1.5.

It has been found that the odour control material can be incorporated into the fibrous structures according to the present invention in an amount that ranges from 20 g/m² to 400 g/m², preferably from 100 g/m² to 300 g/m², more preferably from 150 g/m² to 250 g/m², with reference to the total surface area of the fibrous structure. Preferably the fibrous structures of the present invention comprise from 20% to 80% by weight of the odour control means. The weight of the odour control means that can be actually used in various fibrous structures intended for different uses can be readily determined by the skilled person bearing in mind the size and the type of the fibrous structure, and its intended use.

The latex is applied as an aqueous emulsion or dispersion, which typically contains about 45% to 65% solids, and these materials are readily available from several manufacturers. Because the latex emulsions are water miscible, they may be further diluted, if desired, before being applied to the web. Also, these latex compositions are thermosetting, and in order to effect cross-linking, they contain a small amount of a suitable cross-linking agent which are well known chemical agents for this purpose, such as N-methylolacrylamide. Any type of latex known in the art which is suitable for fibrous structures of the present invention can be used, provided that it does not generate detectable odours, especially after curing, since this could exhaust at least partially the odour control capacity of the fibrous structure well before its intended use. Latices available are classified by chemical family, and those particularly useful include vinyl acetate and acrylic ester copolymers, ethylene vinyl acetate copolymers, styrene butadiene carboxylate copolymers, and polyacrylonitriles, and sold, for example, under the trade names of Airbond, Airflex and Vinac of Air Products, Inc., Hycar and Geon of Goodrich Chemical Co., and Fulatex of H. B. Fuller Company. The amount of latex used in the absorbent structure cannot be so high as to substantially impair or obscure the effective odour control capacity of the odour control means, and also the absorbent properties of the hydrophilic fibres, or as to impart a stiffness to the structure as to render it impractical. It has been found that the latex may range from about 5% to 30% by weight of the structure, and preferably from about 10% to 20% by weight.

Fibrous structures made according to the present invention exhibit a good integrity due to the latex coating, and yet have a remarkable odour control capability, combined in a preferred embodiment with a good absorbent capacity towards aqueous fluids, since the odour control particles are affected by the latex binder only to a minimum extent, owing to the control exerted onto the application of the latex coating to the web in order to form the fibrous structure of the present invention. The depth of penetration of the latex binder into the fibrous web is controlled by the vacuum applied by means of the suction boxes positioned in correspondence with the dispensing means, and by the choice of the amount to be applied to the web.

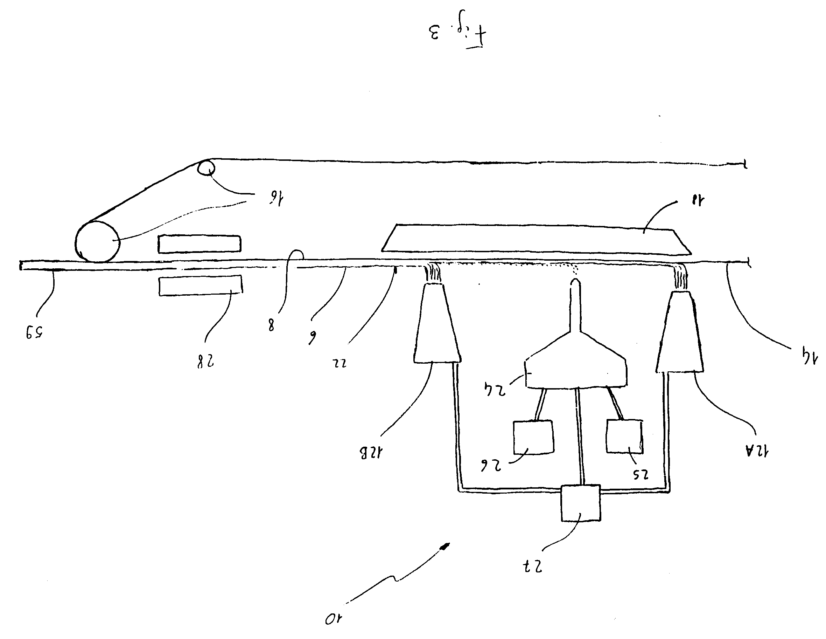

In an alternative embodiment of the fibrous structure of the present invention, being further capable of absorbing aqueous fluids, a porous reinforcing sheet such as a creped paper, a tissue, or a nonwoven, can be incorporated into the fibrous structure either as a surface sheet or as an intermediate sheet disposed intermediate the first and second surfaces of the fibrous web. Referring to FIG. 3, the absorbent fibrous structure 59 comprises fibres 60 and particles 61 of an odour control means interspersed in the web 22, but more concentrated in the middle of the thickness of the structure. The sheet 62 is present on one surface of the web 22, while the opposite surface bears a cured latex coating 64.

The absorbent fibrous structure illustrated in FIG. 3 can be formed on an apparatus similar to that illustrated in FIG. 1, with some modifications that can be determined by the skilled person. In an embodiment the sheet 62 can be a polyester or a polyolefin nonwoven onto which the fibres can be layered by the fibre distributor, a bonding between the fibres and the sheet being achieved by means of mechanical entangling, while the latex binder is subsequently only applied to the surface of the web which is opposite to the sheet 62.

## Claims

1. A dry laid fibrous structure for providing odour control, said structure comprising a dry laid fibrous web having a first surface and a second surface aligned approximately opposite to said first surface, and further comprising a latex coating on at least one of said surfaces of said fibrous web, said dry laid fibrous structure being characterized in that it further comprises an odour control means for controlling malodours.

2. A dry laid fibrous structure according to claim 1, characterized in that said odour control means comprises one or more odour control agents.

3. A dry laid fibrous structure according to any preceding claim, characterized in that said odour control means is in particle or powder form.

4. A dry laid fibrous structure according to any preceding claim, characterized in that said odour control means is distributed within the thickness of said dry laid fibrous web, intermediate said first and said second surfaces.

5. A dry laid fibrous structure according to any preceding claim, characterized in that said dry laid fibrous structure comprises from about 5% to 30% by weight of latex, preferably from 10% to 20%.

6. A dry laid fibrous structure according to any preceding claim, characterized in that said dry laid fibrous structure comprises from 10% to 80% by weight of said odour control means.

7. A dry laid fibrous structure according to any preceding claim, characterized in that said dry laid fibrous structure is preferably air laid.

8. A dry laid fibrous structure according to any preceding claim, characterized in that said dry laid fibrous structure is an absorbent structure for further providing absorption of fluids, particularly aqueous body fluids.

9. A disposable sanitary article comprising a dry laid fibrous structure according to any preceding claim, preferably as absrbent core
